# EUROPEAN PATENT APPLICATION

(11) **EP 4 279 068 A1**
(43) Date of publication of application: **22.11.2023**
(21) Application number: 23173726.3
(22) Date of filing: 16.05.2023
(51) Int. Cl.: A61K 31/122, A61K 31/165, A61K 36/05, A61K 8/35, A61K 45/06, A61P 17/00, A61P 17/06, A61P 17/10, A61Q 19/08

(54) **COMPOSITION COMPRISING A COMBINATION OF CAPSAICIN AND/OR PHENYLCAPSAICIN AND ASTAXANTHIN FOR THE PREVENTION AND TREATMENT OF INFLAMMATORY-BASED SKIN DISEASES, IN PARTICULAR ASSOCIATED WITH SKIN AGING**

(30) Priority: 16.05.2022 IT 202200010115
(71) Applicant: Safi Medical Care s.r.l., 50129 Firenze (IT)
(72) Inventor: COCCOLONI, Stefan, 8700 küsnacht (CH); SCAPAGNINI, Giovanni, 95131 Catania (IT); DAVINELLI, Sergio, 86100 Campobasso (IT); PORCARO, Piercarmine, 50129 Firenze (IT)
(74) Representative: Zaccaro, Elisabetta

(57) **Abstract**

The present invention concerns a composition containing capsaicin or phenylcapsaicin in combination with astaxanthin which are dispersed in a physiologically acceptable vehicle and a food supplement containing the composition. The supplement finds application in the prevention and treatment of inflammatory-based skin diseases in particular associated with skin aging such as inflammaging.

## Description

### FIELD OF THE INVENTION

The present invention concerns a combination composition in the prevention and treatment of inflammatory-based skin diseases in particular associated with skin aging.

The present invention originates in the sector of products for treating forms of skin inflammation orally or topically.

In particular, the present invention concerns a food supplement for oral administration or a topical composition suitable for treating inflammatory-based skin diseases, and skin conditions correlated to skin aging such as inflammaging.

### Prior art

The skin represents the most extensive tissue of the human organism and since it develops towards the outside of the human body it performs a barrier function.

Its continuous exposure to external agents, mainly ultraviolet rays, smog, atmospheric agents and microorganisms present in the environment, causes a faster aging thereof and in any case more evident than the other tissues of the human body.

The prolonged exposure to external agents weakens the skin's local immune response promoting conditions in which diseases with an inflammatory component develop. It is widely recognized in the scientific literature that the etiopathogenesis of some of the most common skin diseases such as psoriasis, atopic dermatitis, rosacea, acne, despite being multifactorial, still has a strong inflammatory component.

These dermatological diseases make more evident the signs of skin aging such as wrinkles and facial wrinkles, in the adult or elderly population.

The inflammatory component is quite common in dermatological diseases or in milder skin conditions.

It has been observed that numerous skin diseases begin with an acute inflammatory process. This phenomenon represents a defensive mechanism that the body puts in place, transiently, and that is activated in the presence of tissue damages related to the exposure of agents of various kinds such as pathogens like bacteria, viruses, ultraviolet radiations or by contact with irritants.

When the inflammatory state persists and the inflammation becomes chronic like, for example, in the case of recurrent skin diseases such as atopic dermatitis and psoriasis, some typical local manifestations like redness, itching, pain and skin thickening become evident.

These skin manifestations can exacerbate skin aging and in the long run lead to the onset of a clinical condition known as inflammaging. This clinical condition is the result of a persistent chronic physiological stimulation of the innate immune system that becomes less effective and is damaged as the human body ages. It is a chronic inflammatory state caused by repeated immune reactions to antigens that can generate not only limited damages to the skin and thus represents a general risk factor for the health of the individual even in the absence of clinical signs or symptoms.

The diffusion of this condition within the human body is quite wide and can also be attributed to the action of Micro RNA (MiR), small endogenous molecules of singlestranded non-coding RNA found in the transcriptome. These are polymers encoded by the nuclear eukaryotic DNA about 20-22 nucleotides long mainly active in the regulation of gene expression at the transcriptional and post-transcriptional level. Micro RNAs play a key role in regulating gene expression and are involved in regulating the inflammatory cascade.

In particular 3 of some of these molecules, known as MiR 21, MiR 126 and MiR 146a or inflammo-miRs, exhibit a significant pro-inflammatory activity.

The inflammo-miRs specifically target the NFkB pathway: a protein complex that fundamental in regulating the immune response to infections.

This pathway represents a form of cellular defence that regulates the inflammatory processes caused by oxidation and is directly involved in the aging processes of the cells and the human body. The plasma levels of these MiRs vary with age and with the presence of inflammatory pathologies.

At the cutaneous level, this pro-inflammatory process is added to a progressive degradation of the collagen fibres and to the oxidation action of the epidermal cells caused by exposure to external agents. The combined action of these factors predisposes to the development of skin diseases with an inflammatory component and to an early aging of the skin, especially of the face with accelerated formation of wrinkles.

Faced with this situation, there is a demand for nutritional products that carry out a local anti-inflammatory action and that consequently prevent or slow down the formation of the skin signs typical of aging.

However, the majority of the anti-aging nutritional products on the market does not have a specific activity against inflammaging.

In light of the foregoing, one of the objects of the present invention consists in providing a composition or a supplement containing the same that finds application in the prevention and/or treatment of diseases or conditions of the skin with an inflammatory component.

Another object of the invention is to provide a composition containing a combination of components of natural origin which is suitable for treating the inflammatory component of skin diseases and reducing the skin inflammatory states.

A further object of the present invention consists in providing a composition or a food supplement for treating or reducing the inflammaging and/or slowing down the associated typical signs of skin aging.

### SUMMARY OF THE INVENTION

As part of the activity of research for and screening substances with anti-inflammatory activity, the inventors observed that two selected substances, when combined, exert a synergistic antioxidant and anti-inflammatory effect on a specific biological model.

By analysing the biological mechanisms that contribute to the cellular oxidative stress and to inflammation in the human body, the inventors focused their research on the Nrf2 factor, a member of the Cap'n'Collar family of transcription factors. Nrf2 is sequestered in the cytoplasm by binding to the Kelch ECH protein that associates protein 1 (Keap1) under unstimulated conditions. In turn, Keap1 plays a central role in regulating the response of Nrf2. Under physiological conditions, Nrf2 is targeted by Keap1, which promotes proteasomal degradation of Nrf2 by interactions with a ubiquitin ligase. Keap1 also functions as a stress signal sensor, through stressinduced oxidation of key cysteine residues that lead to conformational changes and the inability to bind Nrf2.

It has been observed that some stimuli, including oxidative stress and the electrophilic compounds, such as some polyphenols, lead to the breakdown of the Nrf2/Keap1 complex, releasing Nrf2 for translocation to the nucleus where it interacts with basic leucine zipper transcription factors such as the members of the Maf and Jun families and binds to a cis-agent element, the antioxidant response element known as ARE, also referred to as EpRE or OSRE.

Nrf2-ARE is a major pathway that regulates phase II antioxidant responses, triggering the simultaneous expression of numerous protective enzymes and scavengers. ARE is found in several cytoprotective inducible genes correlated to the so-called cellular stress response, such as glutathione-S-transferase, heme oxygenase 1 (HO-1), glutamate cysteine ligase, ferritin, gamma glutamyl cysteine synthetase, NAD(P)H quinone oxidoreductase thioredoxin, and pyridoxines, and this emphasizes Nrf2 as a central node in the cell survival response.

With the constant activation of this complex genetic mechanism, it is therefore possible to gradually repair all damages as they occur.

On the basis of such evidence the inventors have addressed the modulation of Nrf2 and HO-1 in the skin in order to test compounds for use in the prevention and/or treatment of skin disorders with inflammatory component in particular connected with skin aging as in the case of inflammaging.

Among the compounds identified, it was observed that capsaicin or phenylcapsaicin and astaxanthin modulate Nrf2 and unexpectedly, when combined, cause a synergistic action.

In accordance with one aspect, the present invention provides a combination composition comprising capsaicin or phenylcapsaicin and astaxanthin and a physiologically acceptable vehicle and/or excipient for preventing and treating a skin disease with inflammatory component.

The combination composition described herein is particularly suitable for treating skin diseases with inflammatory component that influences and/or accelerates the skin aging process as a result of the acceleration of cell degeneration processes e.g. inflammaging.

The combination of capsaicin (CAP) or phenylcapsaicin and astaxanthin (ASX) unexpectedly showed the ability to synergistically activate the nrf2 transcription factor system and the genes correlated thereto, and to down-regulate the NF-kβ activation and the pro-inflammatory cytokine expression, thereby increasing the cellular defences and reducing both the oxidative stress and the inflammatory processes.

These biological activities result in the promotion of biological processes of prevention of the deterioration and death of the epidermal cells caused by degenerative phenomena, photo-aging and by the natural aging process of the human body.

It has also been observed that the combination of capsaicin or the derivative/analogue thereof phenylcapsaicin with astaxanthin performs a stimulating action of the biological mechanisms that slow down the expression of the secretory phenotype associated with senescence, contributing to delaying and forming in the microenvironment an inflammatory response associated with skin aging and the decay of cognitive functions.

In general, the combination composition described herein finds application in the prevention or treatment of skin diseases with an inflammatory component, in particular associated with premature aging.

In particular, the combination composition described herein is for use in the treatment of skin diseases with inflammatory component such as inflammaging, dermatitis, atopic dermatitis, rosacea, seborrheic dermatitis, eczema, or folliculitis, preferably in the treatment of inflammaging accompanied by skin aging and/or signs thereof such as wrinklings, wrinkles, skin folds.

The term Inflamm-aging refers to a chronic, asymptomatic, low-grade inflammation that occurs in the absence of infection in later life. This chronic inflamed state has harmful effects on skin health and contributes to the biological aging and to the appearance of signs of skin aging.

According to some embodiments, the composition of the invention is a pharmaceutical composition or a food supplement containing the same and that can be introduced into the food regimen of an individual in need of treatment. According to certain embodiments the composition described herein is a topical composition to be applied to the skin in the area of the body of therapeutic interest. Typically, in the composition or supplement of the invention the biologically active ingredients capsaicin or phenylcapsaicin and astaxanthin are available in a nutraceutical, or dietary pharmaceutically effective amount.

The present invention will be described in detail below with reference to the following figures.

### BRIEF DESCRIPTION OF THE DRAWINGS

Some features and advantages of the present invention will be apparent from the enclosed figures wherein:

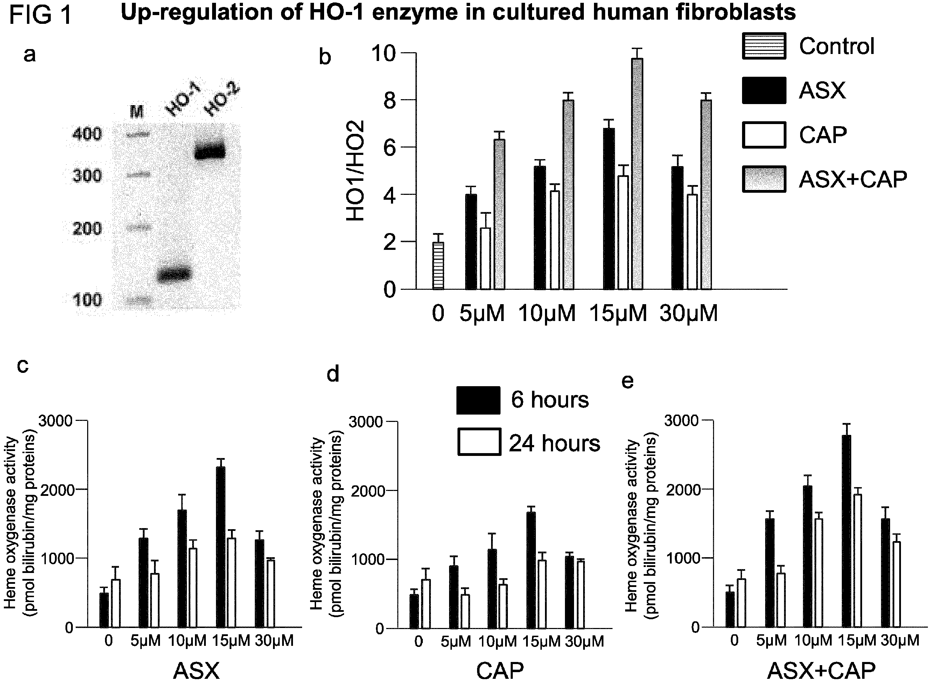

Figure 1 illustrates bar graphs attesting the synergistic effect of a treatment with a composition containing capsaicin (CAP) and astaxanthin (ASX) on the HO-1 mRNA expression and on the enzyme activity in cultured human fibroblasts, according to Example 3.
Figure 2 illustrates a preferred relationship between the two components of the formulation, in order to activate HO-1, according to Example 3.
Figure 3 shows bar graphs illustrating the time-dependent increase in Nrf2 protein expression in the nuclear extracts of Example 3, after treatment with CAP and ASX alone and in combination.
Figure 4 shows graphical bars illustrating the lower NF-kβ DNA-binding activity observed in the macrophages with LPS stimulation in the presence of a combination of CAP and ASX according to Example 4.
Fig. 5 shows bar graphs illustrating the synergistic effect of a composition containing CAP and ASX on the inflammatory cytokine secretion in cultured macrophages, according to Example 4.

### DETAILED DESCRIPTION OF THE INVENTION

The Applicant has found that the administration or topical application of a combination composition described herein has a protective effect on the degenerative processes induced by inflammation, cellular oxidative stress and aging in particular of the skin.

The combination composition of the invention has both a preventive and a treatment action of the skin diseases with inflammatory component.

The inventors found in experimental models, the ability of capsaicin or phenylcapsaicin to increase, with an effect of unexpected intensity, the ability of astaxanthin to activate the Nrf2 pathway and to consequently express defensive and reparative genes, such as HO-1, and improve the intracellular amount of endogenous glutathione.

It has been hypothesized that this effect is due to the fact that capsaicin or phenylcapsaicin share with all other classes of phase 2 inducers, such as electrophilic compounds, the ability to react with sulfhydryl groups which are the specific sensors for Nrf2 activation. However, these effects are surprisingly enhanced when capsaicin or phenylcapsaicin are combined with astaxanthin.

It has also been observed that said compounds perform a synergistic action of inhibition of the Nf-kβ activity and of reduction of the cytokine expression, in an in vitro inflammation model.

In accordance with one aspect there is then provided a combination composition as defined in the appended claim 1.

In other aspects of the invention there is provided a combination composition for the uses defined in the appended claims 6-10.

The protective and anti-aging activity of capsaicin or phenylcapsaicin and astaxanthin and a mixture of the two compounds are experimentally evidenced by the tests of Examples 4 and 5.

The biological model used by the Applicant experimentally demonstrates the synergism of action of capsaicin or phenylcapsaicin combined with astaxanthin in preventing the ROS (reactive oxygen species)-induced damage or, more generally, the damage-correlated cell death since each of them activates a cellular response pathway to injury. More specifically, the combination composition has an ability to synergistically activate the nuclear transcription factor Nrf2. These effects underlie the antiaging and anti-inflammaging use on the skin of the combination composition as described herein.

Nrf2 interacts with the ARE sequence at the promoter level of several inducible genes with critical defensive and reparative properties, resulting in a cellular overexpression of these genes, an improvement of the cell defences, and an inhibition of cell death. Electrophilic phytochemicals have been shown to activate the Nrf2/ARE pathway, including ASX (Davinelli et al 2022).

Within the scope of the present invention, the measurement of the antioxidant activity can be carried out with the method "Oxygen-Radical Absorbance Capacity" (ORAC)(Cao, G.; et al, (1993) Free Radic. Biol. Med. 14(3):303-11), or with the method Total Radical-Trapping Antioxidant Parameter (TRAP) (Ghiselli, A.; et al (1995) Free Radic. Biol. Med. 18(1):29-36; Wayner, D. D et al (1985) FEBS Lett. 187(1):33-7) which determine the ability of the substances to retard or quench ROS produced by free radical generators.

In accordance with one aspect of the invention, the combination of capsaicin or phenylcapsaicin with astaxanthin exerts an anti-inflammatory and synergistic antioxidant effect on the skin that contributes to the anti-inflammatory effect and to a reduction of the skin aging signs, connected to the inflammatory state.

In addition, the administration of the combination composition improves the appearance of the skin promoting the renewal of the cells of the deep and intermediate layers of the epidermis and increasing the elasticity thereby reducing the depth of wrinklings and wrinkles while providing a rejuvenating effect.

The found effect is synergistic and makes the combination composition suitable in the prevention or treatment of skin diseases with an inflammatory component and/or in skin aging characterized by the presence of signs on the skin, for example, facial wrinkles, skin wrinkles, wrinkling, thickening of the integument, loss of elasticity.

One of the biologically active ingredients of the combination composition is astaxanthin or the ester thereof. Astaxanthin belongs to the broad class of the terpene compounds and chemically is a keto-carotenoid having the chemical name of 3,3'-dihydroxy-β-carotene-4,4'-dion and having the following formula

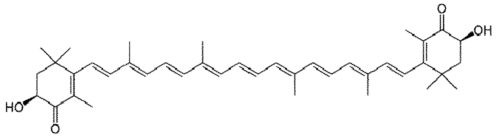

Astaxanthin belongs to the family of xanthophylls, carotenoids that contain oxygen atoms on the terminal rings. The chemical structure with 4 oxygen atoms, in particular an hydroxyl and a ketone for each of the two ends makes astaxanthin a xanthophyll with two specific characteristics:
- amphipathic character (hydrophobic in the central polyene chain and hydrophilic at both ends). This feature favours the positioning of the molecule transversely to the phospholipid double layer of the cell membranes;
- high antioxidant power. This property can be attributed to the deactivating power towards the radicals of oxygen (ROS), due to the coordinated action of the conjugated double bonds and of the ketone groups.

Astaxanthin contains two chiral centres, each of which can assume two stereoisomeric conformations, for a total of 3 stereoisomers 3R,3'R and 3R,3'S meso and 3S,3'S.

In nature, the 3S-3 'S form is most commonly represented.

Synthetically produced astaxanthin is a mixture of the three different forms.

For example, the synthetically produced astaxanthin contains mixtures of the three stereoisomers, for example in the proportions 1:2:1.

Astaxanthin can exist in two main forms, the unesterified and the esterified form, for example 60-90% mono- and 10-30% di-ester, in particular 80% mono- and 20% di-ester.

Advantageously, astaxanthin is contained or extracted from an algae.

Suitable natural sources of astaxanthin are selected from the group of algae or microalgae including Euphausia pacifica, Euphausia superba, Haematococcus pluvialis, Pandalus borealis, Xanthophyllomyces dendrorhous, formerly known as Phaffia rhodozyma.

In particular, the microalga Haematococcus pluvialis contains the highest levels of astaxanthin and is the major source for the non-synthetic production of astaxanthin and preferred herein. Astaxanthin can be produced and obtained from natural sources, such as algae, by conventional technologies e.g. as described in U.S. patent No. 6,022,701 in the name of Boussiba; Sammy, V.; Avigad, C.; et al. (2000), entitled Procedure for large-scale production of astaxanthin from haematococcus, in which it is described: "A process for cultivating Haematococcus for the large scale production of astaxanthin-enriched Haematococcus cells comprises: (a) cultivating said Haematococcus cells under conditions suitable for optimal vegetative growth of said cells, wherein said conditions comprise growing the cells under a light intensity in the range of about 30-140.mu.mol photons.m⁻².S⁻¹ and at a temperature of between about 15-28.degree; and (b) collecting the cells grown according to (a) and cultivating them further under conditions suitable for optimal induction and accumulation of astaxanthin in said cells, wherein said conditions comprise inoculating said cells of (a) into a growth solution containing essentially a carbon source and growing said cells at a temperature of below 35 degree."

A suitable source of astaxanthin for the uses of the invention is from ground algal biomass.

By way of example, the extraction of a suitable astaxanthin from algae or microalgae provides that the collection of the aplanospores is carried out at the end of the green phase, when the level of chlorophyll is at a minimum, using as a source the microalga *H. pluvialis.* The biomass of *H. pluvialis* is dried, ground and standardized, e.g. an astaxanthin content from 1 to 10%, preferably at 4-6%.

Alternatively astaxanthin can be purified, extracted as 8-12% astaxanthin standardized oleoresin by conventional technology e.g. with supercritical CO₂, or as a powder containing from 1 to 5% astaxanthin.

In some embodiments, the composition of the invention contains astaxanthin in an amount ranging from 0.01 to 100 mg, from 0.3 to 30 mg, from 1 to 10 mg per dosage unit, e.g., per capsule.

In certain embodiments the composition of the invention contains astaxanthin in an amount ranging from 0.05 to 35% by weight, from 0.1 to 20% by weight, from 1 to 12% by weight, with respect to the total weight.

In the combination composition of the invention astaxanthin is combined with capsaicin or a derivative or analogue thereof such as phenyl capsaicin, having the following structure formula:

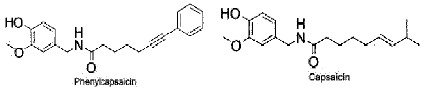

*Capsaicin* known by the IUPAC name of (*E*)-*N*-(4-hydroxy-3-methoxybenzyl) -8-methylnon-6-enamide is an irritant compound found in plants of the genus Capsicum, typically chili pepper.

Capsaicin is present in the placental tissue that supports the seeds, in the inner membranes and, to a lesser extent, in the fleshy parts of the fruits of the plants of the genus Capsicum, in particular *Capsicum annuum.*

The seeds do not produce capsaicin, which is found in high concentration only in the white inner wall, where the seeds are attached.

In the combination composition as an alternative to capsaicin it is possible to use phenylcapsaicin, a derivative or analogue of capsaicin produced by a synthesis process. Compared to natural capsaicin, phenylcapsaicin has a phenylethynyl group on the acyl chain.

In particular, a suitable phenylcapsaicin (N -[(4-hydroxy-3-methoxyphenyl)methyl]-7-phenylept-6-inamide, C₂₂ H₂₃ NO₃, CAS No.: 848127-67-3) has a molecular weight of 337.41 Da, a water solubility of 0.00193 g/L, a partition coefficient (n-octanol/water) of 2.34. The pH is 6.12 at 25°C and has a density of 1.152 g/cm3 at 20°C.

A suitable synthesis process comprises two steps. The first step comprises the production of the acetylenic acid intermediate through the reaction of phenylacetylene with a carboxylic acid derivative. The second step comprises reactions of the acetylenic acid intermediate with the vanillylamine derivative to produce the final product.

In certain embodiments, phenylcapsaicin is formulated with cellulose, fats and/or oil prior to use at a final concentration of 1-1.5%.

The absence of severe side effects even following administration of high dosages of phenylcapsaicin makes it suitable for the uses of the invention.

In accordance with some embodiments, capsaicin or phenylcapsaicin is present in the composition in an amount from 0.1 to 30% from 0.5 to 20%, from 1 to 10% e.g. 5% by weight with respect to the total weight of the composition.

In some embodiments, the composition of the invention contains phenylcapsaicin in an amount ranging from 0.001 mg to 10 mg, preferably more preferably from 0.5 to 2.5 mg.

For example, the maximum intake of capsaicin or phenylcapsaicin per day is 2.5 mg, which corresponds to 36 µg/kg of body weight per day for adults, considering an average body weight of 70 kg and 58 µg/kg of body weight per day for adolescents, considering an average body weight of 43.4 kg.

The combination of capsaicin or phenylcapsaicin with astaxanthin results in a particularly evident anti-inflammatory synergistic effect on the skin and an antiantinflammaging action.

Within the scope of the present application the term combination is understood to mean that one or more active ingredients are added or mixed with one or more other ingredients.

By the terms "synergism", "pharmacological synergism" and "cellular parameters directly relevant to cellular aging" is meant the definition of biological synergism, deriving from the study of the action of drugs.

Drug synergism occurs when two drugs interact in ways that potentiate or amplify one or more effects, or side effects, thereof. In other words, two drugs that produce openly similar effects will sometimes produce exaggerated or diminished effects when used simultaneously, and a quantitative evaluation is necessary to distinguish these cases from the simple additive action (Tallaride RJ. Drug synergism: its detection and applications. J Pharmacol Exp Ther. Set. 2001;298(3):865-72). The biological effects we are focusing on are the positive modulation of the cellular defensive and repair systems, with a relevant effect on the cell survival and longevity, both in terms of individual cells and cell populations.

The pharmacological definition of synergism, when translated in terms of molecular events, typically refers to either the ability of the two bioactive substances producing synergism to potentiate the cellular functions by eliciting pathways that are originally distinct but converge at higher levels into elementary cellular activities concomitant with the same major cellular program. Therefore, if there are two ways for a cell to avoid death controlled by two not-correlated or minimally correlated pathways A and B, it is likely that potentiation of only A or B by two compounds will produce at the higher doses the saturation of the pathway, and therefore a combined effect that is lower than the additive effect. The complete stimulation of A and B, on the other hand, could produce additivity or even synergism (a) because all the activities that lead to that response are engaged, (b) because the cross-talks that occur where the pathways converge can become mutually potentiating, or, finally, (c) because the synchronous activation of both allows the cell to move in distinct but sequential steps of the activity.

In accordance with some aspects of the invention there is provided a supplement comprising a combination composition in accordance with any one of the embodiments described herein or claimed.

A suitable supplement is a food or dietary supplement or alternatively a nutraceutical one.

In some embodiments, the composition of the invention may comprise one or more additional active substances or ingredients.

The composition of the invention may contain one or more other biologically active agents in particular selected from vitamins, for example vitamin C, vitamin E, vitamin A, vitamin D, vitamin K, one or more vitamins of B group and for example B1, B2, B6, B12 or niacin.

In certain embodiments the composition of the invention contains a vitamin in an amount ranging from 0.001 to 20% by weight, from 0.05 to 10% by weight or from 0.5 to 5% by weight.

According to some embodiments, the composition of the invention further comprises one or more micronutrients and/or minerals e.g. salts of Mg, K, Na, Zn, Fe, Cr, Se, Mn.

The compositions of the present invention comprise any composition made by administering the association of active ingredients of natural origin of the present invention and a physiologically acceptable carrier.

Such compositions are suitable for nutritional, pharmaceutical or dietary use in mammals, particularly in humans.

The term "vehicle" as used herein means an excipient, diluent medium with which the association of biologically active ingredients is administered.

Any suitable vehicle and/or excipient suitable for the form of preparation desired for administration to humans is contemplated for use with the compounds described in the present invention.

Within the scope of this application, the term "physiologically acceptable" is intended to mean edible substances that are approved by the health authorities for use in pharmaceutical, phytotherapeutic, nutritional or food applications.

The composition or food supplement containing it for oral administration may take a wide variety of preparation forms, depending on the desired route of administration. The composition of the invention may be in solid forms.

The compositions in solid form comprise tablets, pills, capsules, powders, granules and suitable controlled dosage forms.

A composition is a supplement in the form of water-soluble granules.

In the case of preparations in solid form, vehicles may be employed, for example one or more of starches, sugars, microcrystalline cellulose, diluents, granulating agents, lubricants, binders, disintegrants and the like.

Typically, the tablets, capsules, pills and the like may also contain a binding agent such as gum tragacanth, acacia, corn starch, or gelatin; excipients such as dicalcium phosphate; a disintegrating agent such as potato starch, corn starch, alginic acid; a lubricating agent such as magnesium stearate; a sweetening agent such as sucrose, lactose or saccharin. If desired, the tablets can be coated by traditional techniques. When the form of the dosage unit is in capsule form, it may contain, in addition to the materials of the type described above, a liquid vehicle such as a grease oil.

In accordance with certain embodiments the composition of the invention contains a cellulose-based excipient that comprises i) organic esters of cellulose for example selected from cellulose acetate, cellulose propionate, cellulose triacetate, cellulose acetate propionate, cellulose acetate butyrate, ii) inorganic esters of cellulose for example selected from nitrocellulose, cellulose sulphate, iii) cellulose ethers selected from a) cellulose alkyl ethers for example selected from methyl cellulose, ethyl cellulose, ethyl methyl cellulose; b) hydroxyalkyl ethers of cellulose for example selected from hydroxyethyl cellulose, hydroxypropyl cellulose, hydroxypropyl cellulose, hydroxyethyl cellulose, hydroxypropyl methyl cellulose, ethyl hydroxyethyl cellulose; c) carboxyalkyl cellulose for example carboxy methyl cellulose, Salts thereof and mixtures thereof. In certain embodiments the cellulose-based excipients are crosslinked with physiologically acceptable crosslinking agents.

In accordance with some embodiments the composition of the invention further comprises one or more additional components, such as additives, filling agents, fillers, stabilizers, emulsifiers, plasticizers, filming agents, wetting agents, thickeners, structurants.

In certain embodiments the composition of the invention comprises as an excipient a hydrogenated fatty acid, preferably having a chain with from 3 to 20 carbon atoms, from 14 to 18 carbon atoms. A typical example of a suitable hydrogenated fatty acid is hydrogenated palm oil.

Several other materials may be present as coatings or materials suitable for changing the physical shape of the dosage unit.

For example, the tablets may be coated with sugar shellac or both. If desired, the tablets can be coated with a gastro-resistant film by standard techniques.

In accordance with some embodiments the composition is a powder, advantageously a flowing powder for example dispersible in water, preferably containing from 0.5 to 10% from 1 to 5% by weight of astaxanthin of natural origin. When the composition is in the liquid form it may be in the form of a suspension, emulsion, solution, oleolite. In these cases the vehicle is liquid and can be selected for example from water, glycols, oils, alcohol and mixtures thereof, for example to form oil/water or water/oil emulsions. According to some embodiments the composition may be in aqueous-based or semi-fluid oil-based liquid form.

For example, the formulations in syrup, elixir or oils may contain, sucrose as sweetening agent, methyl and propylparabens as preservatives, a dye and a flavouring agent such as cherry or orange flavour.

In accordance with some embodiments the composition is an oil or oleolite containing astaxanthin preferably from 1 to 25% from 5 to 20% by weight of astaxanthin.

In the composition there may also be flavouring agents, preservatives, colouring agents and the like.

In some embodiments, the active ingredients contained in the composition of the present invention may be combined or mixed as active ingredients in intimate mixture with a suitable edible vehicle and/or an excipient according to the pharmaceutical and food industry or traditional nutritional techniques.

The compositions for nutritional use may suitably be presented in single pharmaceutical form and prepared by means of any of the methods well known in the pharmaceutical or food technique.

The amount of active compounds in the compositions of the invention is such that a preventive or therapeutically effective dosage will be obtained.

In some embodiments, in the supplement of the present invention, the active ingredients are typically formulated in dosage units, e.g., of tablet or of capsule containing the combination composition. In certain embodiments the dosage unit may contain from 0.01 to 100 mg of each active ingredient per dosage unit for daily administration.

In some embodiments, the formulation will contain amounts of active ingredients that will depend on the severity of the form to be treated and on the additional therapies in progress, on the individual health status, and on the response to the association of the active ingredients.

According to some embodiments, the composition of the invention is a nutraceutical, supplement, a nutritional product or a dietary one.

In further embodiments, the composition of the invention is in a form suitable for topical/local application and may be in solid, fluid or semi-fluid form.

Suitable compositions in the solid form comprise creams, pastes, ointments, gels, dressings for pharmaceutical or cosmetic applications and sticks, make-up.

In accordance with a preferred embodiment, the composition is a cream or gel for topical application.

In certain embodiments, the composition for topical use of the invention is in liquid form, for example in the form of an aqueous-based solution or suspension containing one or more vehicles and/or excipients suitable for topical applications. Suitable topical compositions in liquid form include suspensions, solutions, lotions, gels, emulsions. In the liquid form, the composition may generally contain from about 1 to 99.9% by weight of water.

In some embodiments in the topical compositions in liquid form the water is present in an amount from 5 to 95% by weight. In other embodiments, the water is present in an amount from 10 to 90% by weight.

In some embodiments the topical composition is in semi-fluid form, e.g. of foams or emulsions.

In the composition, it is possible to incorporate one or more excipients typically employed in the base formulation of pharmaceutical formulations for topical use, such as oils, glycerin, emollients, emulsifiers, dispersing agents, in amounts that are typical for the desired use.

In some embodiments, the fluid or semi-fluid composition of the invention may contain lipophilic substances, such as oils, for example hydrogenated castor oil.

According to another embodiment, the composition with topical application further comprises one or more of physiologically acceptable thickeners, solubilizers, preservatives, water, alcohols, glycerin, stabilizers, antioxidants and antibacterials in amounts according to the common pharmaceutical practice.

The composition of the invention can be applied, in an effective amount, directly to the skin in the area to be treated.

The following examples are provided primarily to illustrate the present invention and are not intended to limit the scope of protection as is apparent from the appended claims.

### EXAMPLE 1

Supplement for the prevention and/or treatment of skin diseases with inflammatory component in particular inflammaging having the following formulation:

| | |
|---|---|
| Phenylcapsaicin | 2.5 mg |
| Astaxanthin | 250mg |
| Excipients | 800 mg |

### EXAMPLE 2

Composition for the prevention and/or treatment of skin diseases with inflammatory component, having the following formulation:

| | |
|---|---|
| Capsaicin | 2.5 mg |
| Astaxanthin | 250mg |
| Excipients | 800 mg |

### EXAMPLE 3

Effect of a composition containing capsaicin (CAP) and astaxanthin (ASX) on the Nrf2/ARE pathway in cultured human fibroblasts.

Up-regulation of HO-1 and phase II detoxification enzymes in different cells.

The human immortalized ATCC fibroblasts were exposed for 6 hrs to different concentrations of CAP, ASX, or of a combination of the two compounds (5, 10, 15, and 30 µM). The treatment resulted in a significant increase (p < 0.05) in HO-1 mRNA, as measured by quantitative real-time PCR, with a maximum value of 15 µM. ASX causes a dose-dependent increase in HO-1 mRNA, measured relative to the non-inducible HO-2 paralog gene, which peaks (approximately 6-fold) at 15 µM) and subsequently decreases to 30 µM. CAP, on the other hand, is slightly less active in inducing HO-1 gene expression at the same concentrations and peaks again at 15 µM. The combination of the two compounds demonstrates the maximum activation of HO-1. To confirm that the HO-1 gene expression measured at the mRNA level corresponded to an equivalent increase in the heme oxygenase activity, this activity was measured at the same doses of CAP and ASX 6 and 24 hours after administration.

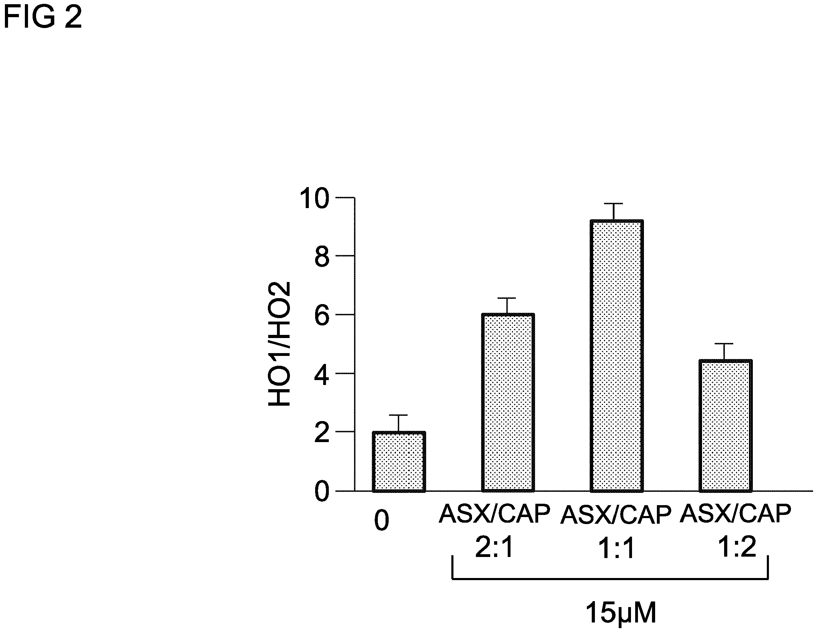

The activity increase pattern was comparable to the results obtained by observing the cellular mRNAs, confirming the functional significance of the data obtained from the real-time PCR determinations. The results highlight the synergistic activity of CAP and ASX in the ability to activate HO-1 gene expression. In order to determine the best ratio between the two compounds we evaluated using the same experimental model, different proportion of the two compounds at the final concentration of 15 µM (CAP/ASX 1:2, 1:1, 2:1). As shown in Figure 2, the 1:1 ratio achieves the maximum effect.

### Activation of Nrf2 expression in different cells.

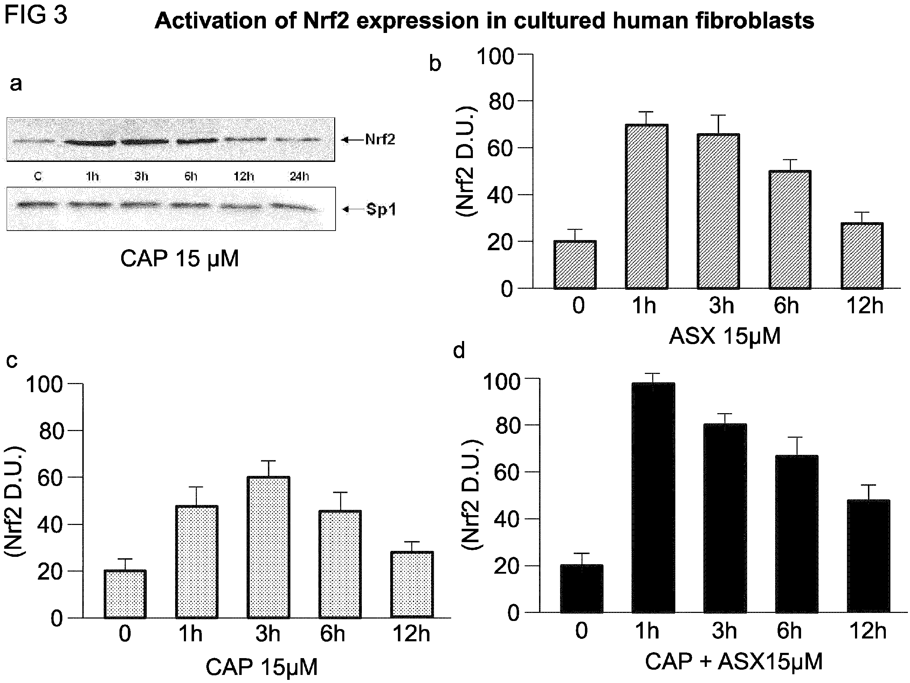

The cultured human fibroblasts were exposed to CAP, ASX or to a combination of the two compounds at the final concentration of 15 µM to evaluate the Nrf2 protein expression over time. As shown in Figure 3, the treatment with ASX caused a significant time-dependent increase in Nrf2 protein expression in the nuclear extracts. A quantification of three independent western blots showed that after 1 hr of exposure to 15 µM of ASX, the Nrf2 expression significantly increased and remained upregulated up to 12 hrs, while the levels of the constitutive transcription factor Sp1 were stable. CAP induced a lower increase in nuclear Nrf2. The combination of the two compounds massively induced an expression of Nrf2.

### EXAMPLE 4

CAP and ASX reduce the NF-kβ p65 DNA-binding activity in the cultured macrophages.

Scientific studies have found that the NF-kβ pathway plays a pivotal role in the etiogenesis of the chronic inflammatory diseases. The family of transcriptional factors NF-kβ plays a critical role in regulating the expression of a wide variety of genes, particularly in the inflammatory process, including cytokines such as IL-1β, IL-6 and TNF-α.

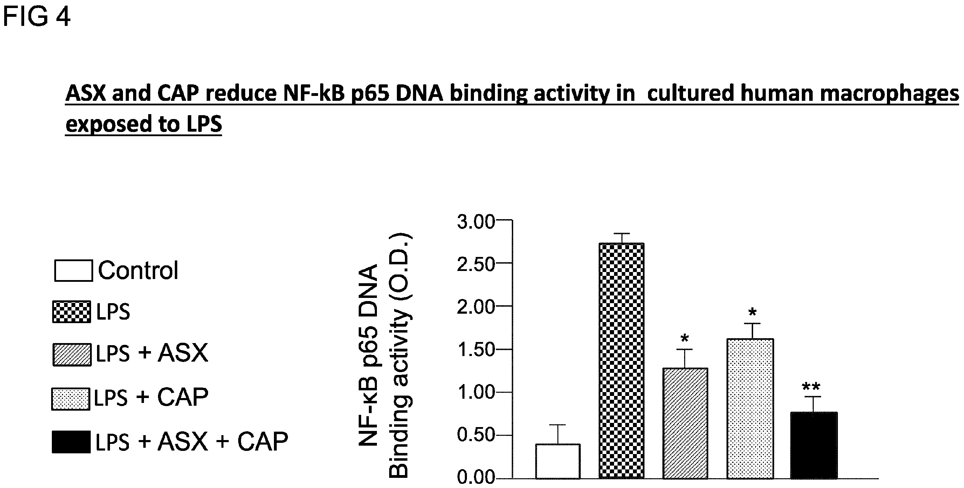

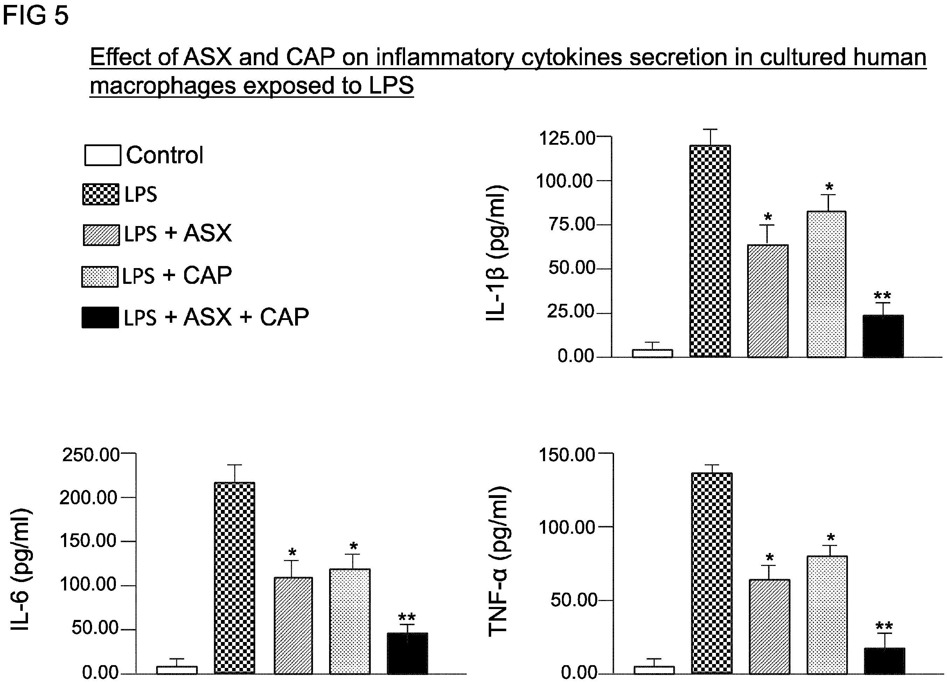

To evaluate the ability of the synergistic composition of the invention to inhibit the NF-kβ activity in macrophages of rats, we used the DNA-binding activity assay of NF-kβ p65 by ELISA kit for TransAM p65 transcription factor (Active Motif, Carlsbad, CA). The cells were stimulated with 500 ng/ml of LPS in the presence and absence of 15 µm of CAP, 15 µm of ASX or a 15 µm combination of CAP + ASX, for 12 hours, according to the invention. Then, the DNA-binding activity of NF-kβ p65 was determined. 12 hours thereafter LPS stimulation markedly promoted the DNA-binding activity of NF-kβ p65 and the expression of proinflammatory cytokines IL-1β, IL-6, and TNF-α. However, a lower DNA-binding activity was observed in the macrophages with LPS stimulation in the presence of both CAP and ASX, as shown in Fig. 4. Surprisingly, the combination of CAP + ASX strongly reduced a DNA-binding activity of NF-kβ p65, demonstrating a synergistic effect obtained by the combination of the two compounds. Consistently, similar effects were shown in terms of cytokine expression (Figure 5). An immortalized macrophage cell line was used. The cells were pretreated or not for 1 hr with 15 µm of CAP, 15 µm of ASX or 15 µm of CAP+ASX, and then exposed to 500 ng/ml of LPS for another 24 hrs. IL-1b, IL-6 and TNF-a were detected by ELISA kit purchased from Abcam, Cambridge, (MA) USA. The data are expressed as mean ±SEM, n = 4; *p < 0.001 vs. LPS, **p < 0.001 vs. CAP or ASX alone.

Firstly, the 24-hour exposure of the macrophages to 500 ng/mL of LPS induced maximal secretion of IL-113, IL-6, and TNF-α, which are all released in small amounts under basal conditions, as shown in Fig.6 the lipopolysaccharide strongly increased the cytokine secretion. The preincubation (1 hr) with 15 µm of CAP or 15 µm of ASX significantly reduced the LPS-stimulated cytokine secretion. Surprisingly, after a 1-hour incubation with a composition with the combination (mixture) of CAP + ASX the secretion of IL-1β, IL-6 and TNF-α was strongly reduced, demonstrating a synergistic effect of the two compounds in the inhibition of cytokine production.

### Additional material

### Heme oxygenase activity assay

The heme oxygenase activity was determined at the end of each treatment as previously described by our group. Briefly, microsomes from harvested cells were added to a reaction mixture containing NADPH, glucose-6-phosphate dehydrogenase,
rat liver cytosol as a source of biliverdin reductase, and the substrate hemin. The mixture was incubated in the dark at 37 °C for 1 hr and the reaction was stopped by the addition of 1 ml of chloroform. After vigorous mixing with vortex and centrifugation, the bilirubin extracted in the chloroform layer was measured by absorbance difference between 464 and 530 nm (_ = 40 m*M*_1cm_1).

### Preparation of nuclear extract and Western blot for Nrf2

The astrocytes were washed twice with PBS 1X. The cells were then harvested in 1 ml of 1X PBS and centrifuged at 3000 rpm for 3 min at 4 °C. The cell pellet was carefully resuspended in 200 ml of cold buffer containing 10 m*M* of HEPES (pH 7.9), 10
m*M* of KCl, 0.1 m*M* of EDTA, 0.1 m*M* of EGTA, 1 µ*M* of DTT, and cocktail complete with protease inhibitors (Roche, Mannheim, Germany).

The pellet was then incubated on ice for 15 min to allow the cells to swell. After this time, 15 µl of 10% NP-40 were added and the tube was mixed with vortex for 10 s. The homogenate was then centrifuged at 3000 rpm for 3 min at 4 °C and the nuclear pellet was resuspended in 30 µl of cold buffer consisting of 20 m*M* of HEPES (pH 7.9), 0.4 *M* of NaCl, 1 m*M* of EDTA, 1 m*M* of EGTA, 1 µ*M* of DTT and protease inhibitors. The pellet was then incubated on ice for 15 min and vortexed for 10 - 15 s every 2 min. The nuclear extract was finally centrifuged at 13,000 rpm for 5 min at 4 °C. The supernatant containing the nuclear proteins was broken down by SDS-polyacrylamide gel and subjected to immunoblot analysis using anti-Nrf2 (1:500 dilution) and anti-Sp1 (1:500 dilution) antibodies.

## Claims

1. A composition in the prevention and treatment of inflammatory-based skin diseases, in particular associated with skin aging and/or inflammaging comprising a combination of capsaicin and/or phenylcapsaicin and astaxanthin, optionally in the form of a physiologically acceptable ester and a physiologically acceptable vehicle.

2. The composition according to claim 1, wherein said astaxanthin is contained or obtained from an alga selected from Euphausia pacifica, Euphausia superba, Haematococcus pluvialis, Pandalus borealis, Xanthophyllomyces dendrorhous, preferably H. pluvialis and mixtures thereof.

3. The composition according to claim 1 or 2, wherein said alga is an algae biomass, preferably in dried and ground form or in powder form.

4. The composition according to any one of claims 1-3, comprising a combination of phenylcapsaicin and astaxanthin, optionally in physiologically acceptable ester form.

5. The composition according to any one of claims 1-4, further comprising a vitamin preferably selected from a vitamin of B group, in particular vitamin B2, B3, B5, B6, B12, vitamin A, vitamin C, vitamin D, in particular D3, and mixtures thereof and optionally a micronutrient or mineral, preferably selected from Zn, Mg, K, Na, Fe, Cr, Se, Mn, Ca and mixtures thereof.

6. The composition according to any one of claims 1-5, for use in the prevention or treatment of a skin disease with an inflammatory component.

7. The composition for use according to claim 6, wherein the skin disease with inflammatory component is psoriasis, atopic dermatitis, acne, rosacea, seborrheic dermatitis, eczema, or folliculitis.

8. The composition for use according to claim 6 or 7, combined with the use in the treatment of skin aging in particular of the face and its signs, in particular wrinkles, skin wrinkles, wrinkling, thickening of the integument, loss of elasticity.

9. The composition for use according to any one of claims 6-8, wherein said skin disease with inflammatory component is inflammaging.

10. The composition for use according to any one of claims 2-5 or for use according to any of claims 6-9, **characterized in that** it is for oral or topical administration.
